Europäisches Patentamt

European Patent Office

Office européen des brevets

(11) Publication number: **0 355 067**

**A1**

## (12) EUROPEAN PATENT APPLICATION

(21) Application number: **89308423.6**

(22) Date of filing: **18.08.89**

(51) Int. Cl.5: **A 61 K 39/395**

(30) Priority: **19.08.88 GB 8819707**
**19.08.88 GB 8819708**

(43) Date of publication of application:
**21.02.90 Bulletin 90/08**

(84) Designated Contracting States:
**AT BE CH DE ES FR GB GR IT LI LU NL SE**

(71) Applicant: **CELLTECH LIMITED**
**216 Bath Road**
**Slough Berkshire SL1 4EN (GB)**

(72) Inventor: **Opal, Steven Michael**
**111 Brewster Street Memorial Hosp,R.I, I.D.Div.**
**Pawtucket Rhode Island 02860 (US)**

**Bodmer, Mark William**
**131 Reading Road**
**Henley-on-Thames Oxon RG91DJ (GB)**

**Riddel, Alastair James**
**"Kia Ora", Lymington Bottom, Four Marks**
**Alton, Hampshire GU34 5AH (GB)**

(74) Representative: **Hallybone, Huw George et al**
**CARPMAELS AND RANSFORD 43 Bloomsbury Square**
**London WC1A 2RA (GB)**

Claims for the following Contracting States: ES + GR.

(54) **Pharmaceutical products for anti-neoplastic therapy.**

(57) An antibody (anti-TNF) to tumour necrosis factor-α (TNF-α), preferably a monoclonal antibody, is used to prevent or ameliorate the side effects associated with anti-neoplastic therapy. Side effects which may be treated include nausea, vomiting, anorexia, diarrhoea, alopecia, malaise, neutropenia, septicaemia and other conditions arising therefrom. The anti-TNF may be used in pharmaceutical products and compositions together with an anti-neoplastic agent and/or an anti-microbial agent.

EP 0 355 067 A1

**Description**

**Pharmaceutical Products for Anti-neoplastic Therapy**

Technical Field

The present invention relates to pharmaceutical products and compositions for anti-neoplastic therapy. In particular, it relates to products and compositions which include an antibody to tumour necrosis factor alpha (TNF) and to the use of such an antibody in anti-neoplastic therapy and in the manufacture of pharmaceutical products and compositions for such use.

Background Art

Antineoplastic agents damage or destroy cells and are used to treat various types of cancer. They offer successful treatment in some conditions and help reduce symptoms and prolong life in others. Patients undergoing treatment with these agents, however, can suffer a large number of undesirable side effects such as malaise, nausea, vomiting, anorexia, alopecia, diarrhoea, mucositis and neutropenia.

Furthermore, neutropenic patients are susceptible to Gram negative septicaemia and the mortality rate associated with such septicaemia remains unacceptably high despite the availability of a wide variety of potent, broad spectrum antimicrobial agents (1,2). Passive immunotherapy utilizing antibody directed against bacterial lipopolysaccharide (endotoxin) has been considered as an approach to supplement antimicrobial agents in the management of the septic patient (3,4). However, the generation of sufficient antibody levels to provide broad cross protection against multiple LPS serotypes has proven problematic, and results of laboratory and clinical studies using anti-core glycolipid immunotherapy have been variable and sometimes contradictory (3, 5, 6, 7).

There is increasing evidence that the inflammatory cytokine, tumor necrosis factor - alpha (TNF) is a principal mediator in the cascade of pathophysiologic events which follow gram negative septicemia, so that it has been proposed that antibodies directed against TNF might provide protection against many of the injurious effects of endotoxin during gram negative sepsis (8, 9, 10). Clinical studies have demostrated, in fact, that TNF levels are elevated in some septic states, such as meningococcemia (11), and human trials with controlled administration of lipopolysaccharide demostrate that serum levels of TNF elevate rapidly in response to LPS administration (12). Experimental studies in animals suggest that anti-TNF antibody protects rodents from otherwise lethal doses of LPS (8) and a baboon shock model demonstrated protective efficacy of an anti-TNF monoclonal antibody following intravenous challenge of a large inoculum of Escherichia coli (10).

The present inventors have now found, using animal models, that antibody against TNF can mitigate of even prevent some of the side effects associated with anti-neoplastic therapy. Furthermore, the inventors have used an animal model for the study of Gram negative sepsis which closely mimics the pathophysiology of bacterial infection observed clinically in patients receiving anti-neoplastic chemotherapy (13) to demonstrate the efficacy of antibodies to TNF in protecting neutropenic patients from the effects of Gram negative infections.

Disclosure of the Invention

According to the present invention there is provided a method for the manufacture of a medicament for use in anti-neoplastic therapy the method involving the use of an antibody to TNF. The invention also provides a method of treatment of a human subject suffering from a side-effect arising from anti-neoplastic therapy which comprises administering an effective amount of an anti-TNF antibody.

The medicament manufactured according to the method of the present invention may be used to prevent or treat any of those physiological conditions which are generally associated with the use of an anti-neoplastic agent. Particular conditions include, for example, those mentioned above: nausea, vomiting, anorexia, diarrhoea, alopecia, malaise, neutropenia, septicaemia and other conditions arising therefrom.

Thus the invention also provides an antibody to tumour necrosis factor $\alpha$ for use in the treatment or prevention of a side effect arising from anti-neoplastic therapy.

The antibody against $\alpha$-tumour necrosis factor (hereinafter referred to as anti-TNF or anti-TNF antibody) for use according to the invention may in general belong to any immunoglobulin class. Thus for example the anti-TNF antibody may be an immunoglobulin G or immunoglobulin M antibody.

The anti-TNF antibody may be of animal, for example mammalian, origin and may be for example of murine, rat, hamster or human origin. The antibody may be a whole immunoglobulin, or a fragment thereof, for example a F(ab')$_2$ or Fab fragment.

The anti-TNF antibody may be polyspecific but is preferably monospecific for a human $\alpha$-TNF. The antibody may be a polyclonal antiserum or a monoclonal antibody. Particularly useful antibodies for use according to the invention include recombinant anti-TNF$\alpha$ antibodies, i.e. anti-TNF antibodies which have been produced using recombinant DNA techniques. Especially useful antibodies of this type are antibodies having an antigen binding site at least part of which is derived from an immunoglobulin from a non-human species, the remainder of the molecules being derived from a human immunoglobulin.

The anti-TNF antibody may be prepared using well-known immunological techniques employing $\alpha$-TNF as antigen. Thus, for example, any suitable host may be injected with $\alpha$-TNF and the serum collected to yield the desired polyclonal anti-TNF antibody after appropriate purification and/or concentration, (for example by affinity chromatography using immobilised α-TNF as the affinity medium).

Alternatively, splenocytes or lymphocytes may be recovered from the α-TNF-injected host and immortalised using for example the method of Kohler et al., Eur. J. Immunol. 6, 511, (1976), the resulting cells being segregated to obtain a single genetic line producing monoclonal anti-TNFα antibodies in accordance with conventional practice. Antibody fragments may be produced using conventional techniques, for example by enzymatic digestion e.g. with pepsin [Parham, J. Immunol., 131, 2895, (1983)] or papain [Lamoyi and Nisonoff, J. Immunol. Meth., 56, 235, (1983)]. Where it is desired to produce recombinant anti-TNFα antibodies these may be produced using for example the methods described in European Patent Specifications Nos. 171496, 173494, 194276 and 239400.

In order to prevent or treat side-effects arising from antineoplastic therapy, anti-TNF antibodies may in general be administered in an appropriate form and amount at any suitable time before or during the therapy and, where necessary, after the therapy has finished. The anti-TNFα antibodies may be administered seperately or together with the antineoplastic agent.

Thus, according to a further aspect of the invention there is provided a pharmaceutical product containing an antibody to TNF and an anti-neoplastic agent as a combined preparation for simultaneous, separate or sequential use in anti-neoplastic therapy.

For example, a pharmaceutical composition may be provided comprising anti-TNF and an anti-neoplastic agent optionally together with a pharmaceutically acceptable carrier, excipient or diluent. The composition is suitably for the prevention or treatment of side effects arising from anti-neoplastic therapy.

Accordingly, the invention also provides the use of anti-TNF and an anti-neoplastic agent in the manufacture of a medicament for use in anti-neoplastic therapy and also provides for the use of anti-TNF and an anti-neoplastic agent together in anti-neoplastic therapy of human patients by the administration of effective amounts of each component.

In a still further aspect of the present invention there is provided a pharmaceutical product containing an antibody to TNF and an antimicrobial as a combined preparation for simultaneous, separate, or sequential use in anti-neoplastic therapy. Such a product is particularly appropriate for the treatment of patients who are suffering from septicaemia associated with a neutropenic condition.

Thus, for example, a pharmaceutical composition may comprise anti-TNF and an antimicrobial, optionally together with a pharmaceutically acceptable carrier, excipient or diluent. The composition is suitably for use in anti-neoplastic therapy.

Accordingly, the invention also provides the use of anti-TNF and an antimicrobial in the manufacture of a medicament for use in anti-neoplastic therapy and also provides a method of treatment of a human subject suffering from or susceptible to a side effect associated with anti-neoplastic therapy the method comprising administering an effective amount of an anti-TNF antibody and an antimicrobial.

In particularly preferred embodiments the pharmaceutical product according to the present invention contains an antibody to TNF, an anti-neoplastic agent and an antibiotic as a combined preparation for simultaneous, separate or sequential use in anti-neoplastic therapy.

For example, a pharmaceutical composition may be provided which contains all three of: an antibody to TNF, an anti-neoplastic agent and an antibiotic, optionally together with a pharmaceutically acceptable excipient diluent or carrier.

In the various aspects of the invention described above the term antineoplastic agent includes any cytotoxic or cytostatic agent, for example alkylating agents (for example nitrogen mustards, nitrosoureas, bulsulphan, cisplatin, and related compounds such as cyclophosphamide) and antimetabolites such as methotrexate, fluorouracil and mercaptopurine. In particular, the antineoplastic agent may be cyclophosphamide.

Where an antimicrobial is employed this may be for example any suitable systemic anti-infective agent, anti-bacterial or anti-fungal for example a pencillin, cephalosporin, carbapenem, tetracycline or amyloglycoside, chloramphenicol, erythromycin, vancomycin amphoterocin or ciprofloxacin.

The compositions of the invention may take any suitable form for administration, and in particular will be in a from suitable for administration by injection, for example by bolus injection or continuous infusion. Compositions for injection may take such forms as suspensions, solutions or emulsions of all or each of the components in oily or aqueous vehicles, and, may contain formulatory agents such as suspending, stabilising and/or dispensing agents. Alternatively, the composition may be in a dry form, for reconstitution before use with an appropriate sterile liquid.

The dose at which the anti-TNF antibody will be administered will depend on the nature of the antineoplastic agent in use and whether the anti-TNF antibody is being used prophylactically or to treat an existing condition arising from antineoplastic therapy. In general, the anti-TNF antibody administered by infusion at a dose in the range 0.1-20mg/Kg one to four times a day.

Where the anti-TNF antibody is used concurrently in a product or composition with an antineoplastic agent, the latter may be used at generally accepted doses. Likewise, where the anti-TNF antibody is used concurrently with an antimicrobial, the antimicrobial may be used at generally accepted doses.

Brief Description of the Drawings

The invention is illustrated below by way of example and with reference to the accompanying drawings of which:

Fig. 1 shows a survival curve comparing anti-TNF monoclonal antibody treated animals (■......■) with saline treated or irrelevant antibody treated animals (●-----●); ∧ indicates administration of intravenous anti-TNF monoclonal antibody, and indicates administration of cyclophosphamide;

Fig. 2 shows a survival curve comparing animals treated with both ciprofloxacin and anti-TNF monoclonal antibody (----------) with animals treated with ciprofloxacin alone (- - - -). ∧ and are as for Fig. 1.

Fig. 3 shows the change in weight with time of rats treated with 50mg/kg i.v. cyclophosphamide (CTX);

Fig. 4 shows the change in weight with time of rats treated with anti-TNF, cyclophosphamide, or combinations of the two.

## Modes for Carrying out the Invention

The invention is illustrated below by way of example only.

## Example 1

### Use of Antibody against TNF to Protect Neutropenic Rats from Lethal Infection with Pseudomonas Aeruginosa

## Materials and Methods

### Test Strain and Reagent

Pseudomonas aeruginosa 12.4.4. was a gift of Dr. A. McManus (USAISR) San Antonio, Texas. The organism which belongs to Fisher-Devlin-Gnabasik immunotype VI was stored in 10% glycerin at -70°C until ready for use. The day prior to oral challenge, the isolate was streaked on trypticase soy agar (BBL Microbiology Systems, Cockeysville, Maryland) and incubated overnight at 37°C. On the following day, the bacteria were suspended in sterile normal saline and adjusted spectophotometrically to a predetermined inoculum size of approximately $10^6$ organisms.

Ciprofloxacin was kindly provided as a gift from Miles Laboratories (West Haven, Connecticut). Injectable cyclophosphamide (Bristol-Myers, Evansville, Ind.) and cefamandole (Eli Lilly, Indianapolis, Indiana) were purchased commercially.

### Monoclonal Antibody Preparation:

A hamster derived anti-murine TNF monoclonal antibody (designated TN3 19.12) which has been shown to neutralize natural rat tumor necrosis factor-alpha (14) was prepared by R. Schreiber (Washington University, St. Louis, Missouri). The antibody was provided at a concentration of 5.75 mg/ml, pH7 and was free of endotoxin with a limulus lysate assay of <0.05 ng/mg of protein.

A second monoclonal antibody L2 3D9 was also used in these experiments and is a hamster derived monoclonal antibody directed against murine recombinant IL2. This monoclonal antibody does not react against natural murine or rat IL2 and is used as an irrelevant monoclonal antibody which was prepared in a similar manner to the anti-TNF monoclonal antibody.

### Animal Model:

The neutropenic rat model has been described in detail previously (13) and was modified slightly in the current study. Female Sprague-Dawley rates which were viral and bacterial pathogen-free and weighed between 100 and 175 gm were obtained from Charles River Breeding Laboratories (Wilmington, Massachusetts). The animals were housed in soild polypropylene cages with filter hoods and allowed to eat and drink ad libitum. Cefamandole was given intramuscularly at a dose of 100 mg/kg beginning 96 hours before bacterial challenge in order to clear the natural gut flora. This dose was repeated 48 and 24 hours before the first bacterial challenge and 24, 72, 120, 168, 216 and 254 hours thereafter.

Cyclophosphamide was given intraperitoneally at a dose of 150 mg/kg at time 0 and again at 72 hours at a dose of 50 mg/kg.

At time 0, 48 and 96 hours, the challenge strain of Pseudomonas aeruginosa was given orally via an orogastric tube. Oral feedings and other manipulations of the rats occurred under light $CO_2$ anesthesia. Blood specimens were obtained 24 hours before the induction of neutropenia and again at 72 hours and 96 hours following the initial dose of cyclophosphamide. Animals were examined daily and underwent necropsy examination within 24 hours after death or following sacrifice at the end of the ten day period of neutropenia. Necropsy cultures were obtained from lung, heart, liver, spleen and cacum. Non-lactose fermenting, oxidase-positive colonies which appeared on MacConkey's agar were further identified by aggulutination reactions with a polyvalent (Pseudomonas aeruginosa antisera set (Difco, Detroit, Michigan)). Histologic sections of lung tissue, cacum and renal tissue were also performed on four lethally infected animals.

Animals were divided into three groups. Animals in the first group received the anti-TNF monoclonal antibody (TN3 19.12) at a dose of 20 mg/kg intravenously at time 0 and 120 hours; animals in the second group received, instead of anti-TNF, an intravenous injection of saline as a control; the third group of animals received the irrelevant monoclonal antibody L2 3D9 as an additional control. Some animals of each group also received the antibiotic ciprofloxacin at a dose of 2.5 mg/kg/day in two divided doses beginning 72 hours after cyclophosphamide and continuing for three consecutive days. Control animals received normal saline at the same time schedule.

### Serum Measurements of Monoclonal Antibody Levels and Other Determinations:

Serum levels of TN3 19.12 were determined using a direct ELISA system using a peroxidase labelled goat anti-hamster IgG antibody (14). Serum TNF levels were determined using a colormetric determination of cytotoxicity to L929 cells as previously described (15). Ciprofloxacin levels were measured using a microbiologic assay on neomycin assay agar (Antibiotic Medium C: BBL Microbiology Systems, Cockseyville, Maryland) as previously described (13). Ciprofloxacin levels were obtained 90 minutes after an oral challenge of ciprofloxacin at a dose of 1.25 mg/kg. Additional serum determinations included blood cultures and WBC determinations.

## Statistical Methods

Differences in proportions were determined using the Chi Squared test. Serum measurements were compared using a two tailed, paired Student's T-Test. Differences were considered statistically significant at p < 0.05 level. Results are expressed at the mean ± the standard deviation unless otherwise stated.

## Results

## Outcome in Control Animals:

Saline treated control animals and animals receiving the irrelevant monoclonal antibody and no other treatment all died within nine days following the initial cyclophosphamide treatment. Animals became overtly ill within 120 hours after receiving cyclophosphamide exhibiting piloerection, lethargy, poor feeding, diarrhoea and facial swelling. Necropsy material revealed multiorgan infection with the challenge strain of Pseudomonas aeruginosa in over 95% of the control animals. One animal died of apparent gastrointestinal hemorrhage. Histologic examination of four animals revealed evidence of pulmonary congestion and interstitial edema of lung tissue without evidence of abscess or acute bacterial pneumonia. cacal specimens demonstrated mild interstitial edema without mucosal ulceration. Renal tissue uniformly demonstrated evidence of acute tubular necrosis.

## Effect of Anti-TNF and Ciprofloxacin Treatment:

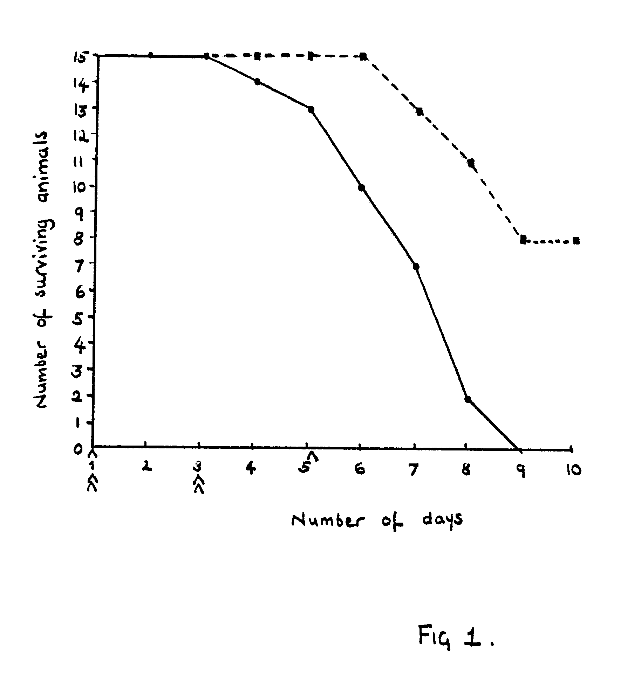

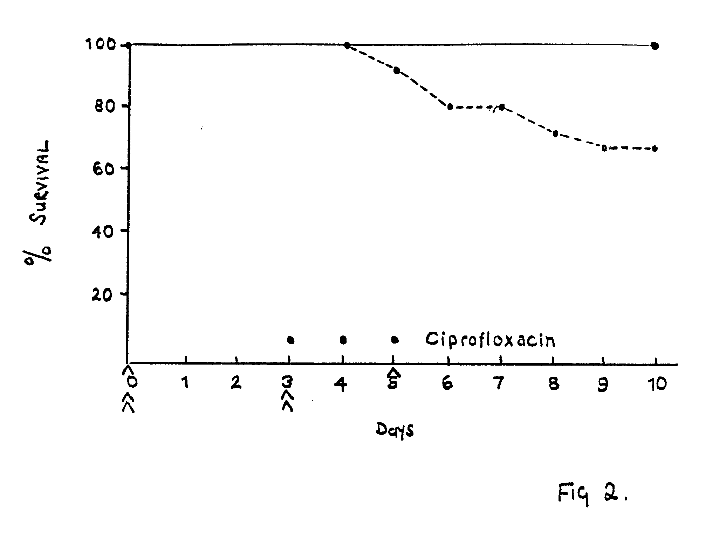

The intravenous administration of the anti-TNF monoclonal antibody protected 8 of 15 animals from lethal infection with Pseudomonas aeruginosa 12.4.4 compared to no survivors in the saline treated or irrelevant monoclonal antibody treated control animals (p < 0.005). The effect of this monoclonal antibody on survival in the neutropenic rat model is depicted in Figure 1. The oral administration of ciprofloxacin resulted in a 67% survival rate (10 of 15 animals) while the combination of ciprofloxacin and anti-TNF monoclonal antibody gave complete protection with 16 of 16 animals surviving the 10 day period of neutropenia (p < 0.05) (Figure 2).

Necropsy material of animals sacrificed at the end of the experiment (11 days following cyclophosphamide treatment) revealed sterile cultures from extraintestinal sites; nonetheless Pseudomonas aeruginosa 12.4.4 was isolated from the cacal cultures.

Random blood cultures performed 24 hours prior to the administration of cyclophosphamide revealed no positive cultures for Pseudomonas aeruginosa. However, random blood cultures obtained 120 hours after the administration of the first dose of cyclophosphamide revealed documented bacteremia in 40% of control animals and 46% of animals receiving anti-TNF Mab (p-NS). None of the ciprofloxacin treated animals had detectable levels of bacteremia with Pseudomonas aeruginosa after 72 hours of oral administration of ciprofloxacin (p < 0.01). Quantitative bacterial cultures demonstrated low level bacteremia measured at 480 ± 44 organisms per ml. The quantitative level of bacteremia was unchanged by anti-TNF Mab when compared with untreated control animals. All blood cultures revealing Pseudomonas aeruginosa were confirmed to be the challenge strain P. aeruginosa 12.4.4. Profound neutropenia occurred in all animals within 72 hours following the administration of the first dose of cyclophosphamide. White blood cell counts were uniformly less than 100 neutrophils per cubic millimeter. Previous experiments demonstrated that neutropenia persists up to 10 days following the administration of cyclophosphamide (13).

The results described above indicate that although the anti-TNF antibody alone did not prevent bacteraemia from occurring it did prevent lethality from Pseudomonas aeruginosa in 43% of animals with documented bacteremia. It was also established by the inventors that circulating levels of tumour necrosis factor were much lower in animals who received anti-TNF antibody compared to control animals. The estimated protective efficacy of anti-TNF Mab alone in this animal model of Pseudomonas aeruginosa sepsis was 53%. The result of the experiments described herein indicate that anti-TNF monoclonal antibody will be useful for preventing mortality in clinical septic shock in neutropenic patients. Furthermore, septic animals in this model developed a characteristic clinical appearance manifested by mucopurulent conjunctivitis, facial swelling, unkempt appearance, piloerection, inactivity, poor feeding, and decreased water uptake. These clinical findings occur at about the third day of neutropenia and persist for five to seven days. Although animals given anti-TNF became bacteremic they did not appear clinically ill.

Clearly when an antimicrobial agent was administered together with anti-TNF additional protective benefit was obtained.

## Example 2

Further experiments were carried out to show that anti-TNF exerted a protective effect against the side effects of anti-neoplastic therapy even where no bacterial infection was induced or anti-microbial employed.

Male Sprague Dawley rats of initial weight 115-130g were employed for these experiments and provided with food and water ad libitum throughout.

In a preliminary experiment eleven rats were used to assess the effect that administration of cyclophosphamide alone had on the animals. A first group of rats (4 animals) were given 50mg/kg intravenously, a second group (4 animals) were given 200mg/kg intravenously, and the third, control, group (3 animals) were given only 0.5ml distilled water.

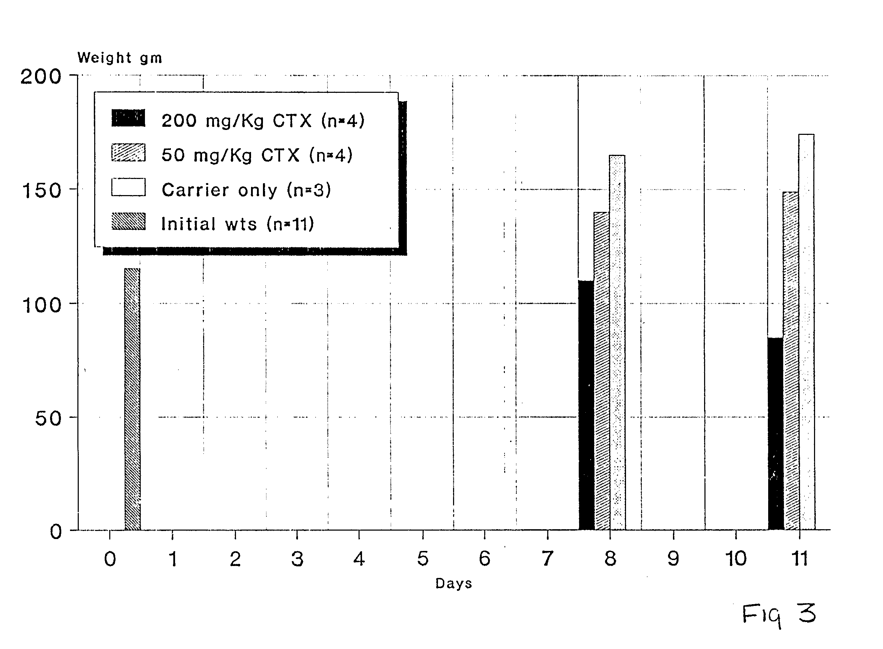

Fig. 3 shows the average weight of each group of animals on days 0, 8 and 11 of the experiment. It is apparent that those animals which were given only distilled water showed a steady increase in weight. The group receiving 50mg/kg of cyclophosphamide also showed an increase in weight but this was less pronouced than in the control group. The average weight of those animals receiving 200mg/kg cyclopohosphamide decreased over the time course of the experiment. It is not clear whether the observed loss of weight or decrease in rate of weight gain was due to loss of appetite or to metabolic effects.

The animals treated with cyclophosphamide began to show signs of illness at around day 8 of the experiment. In fact, there were two fatalities in the high dose group between days 8 and 11. Animals in the control group showed no signs of illness but by day 8 animals in the cyclophosphamide treated group were warm to touch, sluggish, with eye discharge (more pronounced in the high dose group) and hair loss.

In a second experiment, employing a total of twenty-two rats, the effect of coadministration of cyclophasphamide and the anti-TNF antibody TNF3 19.12 was investigated. Five groups of animals were employed as follows:

Group 1 (4 animals) received 50 mg/kg cyclophosphamide intravenously on day zero; Group 2 (4 animals) received 200mg/kg cyclophosphamide i.v. on day zero; Group 3 (4 animals) received 20 mg/kg of anti-TNF i.v. at day 0 and 5; Group 4 (5 animals) received 50 mg/kg of cyclophosphamide i.v. at the start of the experiment, as well as 20 mg/kg anti-TNF at day 0 and 5; and Group 5 (5 animals) received 200 mg/kg cyclophosphamide at the start of the experiment and 20 mg/kg of anti-TNF at the start of the experiment and at day 5.

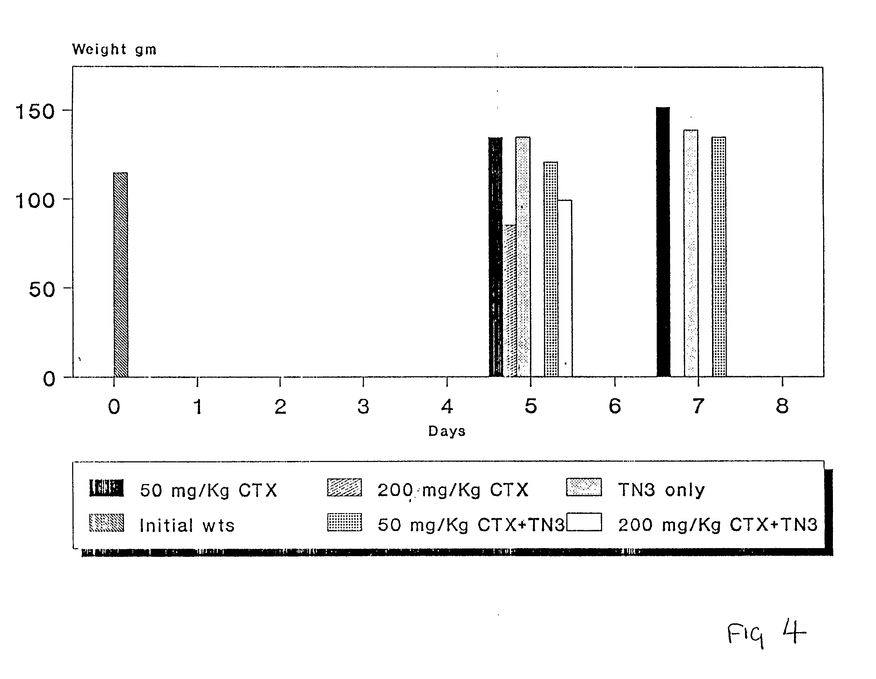

Fig. 4 shows the change in weight of the rats over the first seven days of the experiment. The results were less clear cut than where treatment was with cyclophosphamide alone.

However it is apparent that those rats receiving anti-TNF in addition to a high dose of cyclophosphamide suffered a smaller weight loss over the first five days than did those receiving cyclophosphamide alone. No weights are given for either of the high cyclophosphamide groups at day seven since all of the animals receiving 200 mg/kg cyclophosphamide alone were dead by that stage.

The survival of rats of each group over the time course of the experiment was as follows. All animals in Groups 1, 3 and 4 survived the first seven days of the experiment. Its is noteworthy that of the four animals receiving 200 mg/kg cyclophosphamide alone one had died by day 5 and the rest by day 6. On the otherhand, where the 200mg/kg cyclophosphamide was supplemented by anti-TNF (Group 5) all animals remained alive at day 6 and two out of five survived at day 7.

These results indicated the protective effect of anti-TNF against lethal doses of anti-neoplastic.

At day 5 of the experiment Groups 1, 3 and 4 showed no obvious clinical symptoms from their treatment.

Animals in group 2 were sluggish and showed eye discharge. By contrast, those animals receiving anti-TNF in addition to the high dose of cyclophosphamide (Group 5) showed no eye discharge and were only slightly sluggish, thus demonstrating that anti-TNF treatment ameliorates at least some of the side effects associated with anti-neoplastic therapy.

References

1. Pizzo PA, Young LS. Limitations of current antimicrobial therapy in the immunosuppressed host: Looking at both sides of the coin. Am J Med 1984; 76:S 101-7.

2. Kreger BE, Craven DE, McCabe WR. Gram-negative bacteremia. IV. reevaluation of clinical features and treatment in 612 patients. Am J. Med 1980; 68:344-55.

3. Ziegler EJ, McCutchan JA, Fierer J, Glauser MP, Sadoff JC, Douglas H, Braude Al. Treatment of gram-negative bacteremia and shock with human antiserum to a mutant Escherichia coli. N Engl J Med 1982; 307: 1255-30.

4. Lachman E, Pitsoe SB, Graffin SL. Anti-lipopolysaccharide immunotherapy in the management of septic shock of obstetric and gynaecological origin. Lancet 1984;I:981-3.

5. Greisman SE, Johnston CA. Failure of antisera to J5 R595 rough mutants to reduce endotoxic lethality. J. Infect Dis 1988;157:55-64.

6. Tng NNH, Kaplan HS, Herbert JM, Moore C, Douglas H, Wonderlich A, Braude Al. Protection against gram negative bacteremia and endotoxemia with human monoclonal IgM antibodies. Proc Natl Acad Sci USA 1985;82:1790-4.

7. Ziegler EJ. protective antibody to endotoxin core: the Emperor(s) New Clothes? J. Infect Dis 1988;158:286-90.

8. Beutler B, Milsark IW, Cerami AC. Passive immunization against cachectin/tumor necrosis factor protects mice from lethal effect of endotoxin. Science 1985;229:869-71.

9. Beutler B, Cerami A. Cachectin and tumor necrosis factor as two sides of the same biological coin. Nature 1986; 320:584-8.

10. Tracey KJ, Fong Y, Hesse DG, Manogue KR, Lee AT, Cuo GC, Lowry SF, Cerami A. Anti-cachectin/TNF monoclonal antibodies prevent septic shock during lethal bacteremia. Nature 1987;330:662-4.

11. Girardin E, Grau GE, Dayer J-M, Roux-Lombard P, The J5 Study Group, Lambert P-H. Tumor necrosis factor and interleukin-1 in the serum of children with severe infectious purpura. N Engl J Med 1988;319:397-400.

12. Michie HR, Monogue KR, Spriggs DR, Revhaug A, 0'Dwyer S, Dinarello CA, Cerami A, Wolff SM, Wilmore DW. Detection of circulating tumor necrosis factor after endotoxin administration. N Engl J Med 1988;318:1481-6.

13. Collins HH, Cross AS, Dobeck A, Opal SM, McClain JB, Sadoff JC. Oral ciprofloxacin and a monoclonal antibody to lipopolysaccharide protect leukopenic rats from lethal infection with Pseudomonas aeruginosa. J. Infect Dis 1989;159:1073-82.

14. Sheehan KCF, Ruddle NH, Schreiber RD. Generation and characterization of hamster monoclonal antibodies which neutralize murine tumor necrosis factors. J. Immunol 1989;142:3884-93.

15 Cross AS, Sadoff JC, Kelly N, Bernton E, Gemski P. Pre-treatment with recombinant murine tumor necrosis factor alpha/cachectin and murine interleukin 1 alpha protects mice from lethal bacterial infection. J. Exp. Med 1989;169:2021-7.

## Claims

1. Use of an antibody to tumour necrosis factor-α (anti-TNF) in the manufacture of a medicament for use in anti-neoplastic therapy.

2. Use according to Claim 1 wherein the medicament is for the treatment or prevention of a side effect associated with anti-neoplastic therapy, said side effect being at least one of malaise, nausea, vomiting, anorexia, alopecia, diarrhoea, mucositis, neutropenia, and septicaemia.

3. An antibody to tumour necrosis factor α for use in the treatment or prevention of a side effect arising from anti-neoplastic therapy.

4. A pharmaceutical product containing anti-TNF and an anti-neoplastic agent as a combined preparation for simultaneous, separate or sequential use in anti-neoplastic therapy.

5. A pharmaceutical product according to Claim 4 wherein the anti-neoplastic agent is selected from alkylating agents and anti-metabolites.

6. A pharmaceutical product according to Claim 4 or Claim 5 wherein the anti-neoplastic agent is cyclophosphamide.

7. A pharmaceutical product containing an antibody to tumour necrosis factor-α (anti-TNF) and an antimicrobial as a combined preparation for simultaneous, separate or sequential use in anti-neoplastic therapy.

8. A pharmaceutical product containing an antibody to tumour necrosis factor-α (anti-TNF), an antimicrobial and an anti-neoplastic agent as a combined preparation for simultaneous, separate or sequential use.

9. A pharmaceutical product according to Claim 7 or Claim 8 wherein the antimicrobial is a penicillin, cephalosporin, carbapenem, tetracycline or amyloglycoside, chloramphenicol, erythromycin, vancomycin, amphoterocin or ciprofloxacin.

10. A use according to Claim 1 or Claim 2 or an antibody according to Claim 3 or a pharmaceutical product according to any one of Claims 4 to 9 wherein the antibody is a monoclonal antibody.

## Claims for the following Contracting States: GR, ES

1. Use of an antibody to tumour necrosis factor-α (anti-TNF) in the manufacture of a medicament for use in anti-neoplastic therapy.

2. Use according to Claim 1 wherein the medicament is for the treatment of prevention of a side effect associated with anti-neoplastic therapy, said side effect being at least one of malaise, nausea, vomiting, anorexia, alopecia, diarrhoea, mucositis, neutropenia and septicemia.

3. Use according to Claim 1 wherein a pharmaceutical product is produced by arranging an anti-TNF and an anti-neoplastic agent as a combined preparation for simultaneous, separate or sequential use in anti-neoplastic therapy.

4. A method for the production of a pharmaceutical composition for use in anti-neoplastic therapy, the method comprising mixing anti-TNF and an anti-neoplastic agent and, optionally, a pharmaceutically acceptable, excipient, diluent or carrier.

5. Use according to Claim 3 or a a method according to Claim 4 wherein the anti-neoplastic agent is selected from alkylating agents and anti-metabolites.

6. A method or use according to any one of Claims 3 to 5 wherein the anti-neoplastic agent is cyclophosphamide.

7. Use according to Claim 1 wherein a pharmaceutical product is produced by arranging an anti-TNF and an antimicrobial as a combined preparation for simultaneous, separate or sequential use in anti-neoplastic therapy.

8. A method for the production of a pharmaceutical composition for use in anti-neoplastic therapy, the method comprising mixing anti-TNF and an antimicrobial and, optionally, a pharmaceutically acceptable, excipient, diluent or carrier.

9. Use according to Claim 1 wherein a pharmaceutical product is produced by arranging an anti-TNF, an anti-microbial and an anti-neoplastic agent as a combined preparation for simultaneous, separate or sequential use in anti-neoplastic therapy.

10. A method for the production of a pharmaceutical composition for use in anti-neoplastic therapy, the method comprising mixing anti-TNF and antimicrobial and an anti-neoplastic agent and, optionally, a pharmaceutically acceptable, excipient, diluent or carrier.

11. A method or use according to any one of Claims 7 to 10 wherein the antimicobial is a pencillin, cephalosporin, carbapenem, tetracycline or amyloglycoside, chloramphenicol. erythromycin, vancomycin, amphoterocin or ciprofloxacin.

12. A method or use according to any one of the preceding claims wherein the antibody is a monoclonal antibody.

Fig 1.

Fig 2.

Fig 3

EP 0 355 067 A1

Fig 4

EP 0 355 067 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-260610 (BASF)<br>* page 3, line 37 - page 43 * | 1-10 | A61K39/395 |
| A | EP-A-218868 (NEW YORK BLOOD CENTER)<br>* the whole document * | 1-10 | |
| A | PATENT ABSTRACTS OF JAPAN<br>vol. 10, no. 71 (C-334)(2128) 20 March 1986,<br>& JP-A-60 208924 (ASAHI) 21 October 1985,<br>* the whole document * | 1-10 | |
| A | CHEMICAL ABSTRACTS, vol. 105, no. 4, 28 July 1986<br>Columbus, Ohio, USA<br>LIANG Chi. et al: "Production and characterization of monoclonal antibodies against recombinant human tumor necrosis factor"<br>page 579; column 1; ref. no. 40912 &<br>BIOCHEM.BIOPHY.CHEM.RES.1986,137(2),847<br>* abstract * | 1-10 | |
| A,P | PATENT ABSTRACTS OF JAPAN<br>vol. 13, no. 61 (C-567)(3409) 10 February 1989,<br>& JP-A-63 253099 (SUNTORY) 20 October 1988,<br>* the whole document * | 1-10 | TECHNICAL FIELDS SEARCHED (Int. Cl.5)<br><br>A61K<br>C12P |
| A,P | CHEMICAL ABSTRACTS, vol. 111, no. 5, 31 July 1989<br>Columbus, Ohio, USA<br>SHERRY B. et al: "Anticachetin/tumor necrosis factor-alpha antibodies attenuate developmentof cachexia in tumor models"<br>page 465; column 2; ref. no. 37732 & FASEB J., 1989, 3(8), 1956-62<br>* abstract * | 1-10 | |

The present search report has been drawn up for all claims

| Place of search | Date of completion of the search | Examiner |
|---|---|---|
| BERLIN | 30 OCTOBER 1989 | AVEDEKIAN P.F. |